(19) Europäisches Patentamt
European Patent Office
Office européen des brevets

(11) Publication number: 0 204 287 A2

# (12) EUROPEAN PATENT APPLICATION

(21) Application number: 86107420.1

(22) Date of filing: 02.06.86

(51) Int. Cl.⁴: C 07 C 69/732
C 07 C 67/31

(30) Priority: 04.06.85 US 741069

(43) Date of publication of application: 10.12.86 Bulletin 86/50

(84) Designated Contracting States: CH DE FR GB IT LI NL

(71) Applicant: MERCK & CO. INC.
126, East Lincoln Avenue P.O. Box 2000
Rahway New Jersey 07065(US)

(72) Inventor: Verhoeven, Thomas R.
106 Oak Lane
Cranford New Jersey 07016(US)

(72) Inventor: McNamara, James M.
276 Waite Avenue
Rahway New Jersey 07065(US)

(74) Representative: Abitz, Walter, Dr.-Ing. et al,
Abitz, Morf, Gritschneder, Freiherr von Wittgenstein
Postfach 86 01 09
D-8000 München 86(DE)

## (54) Process for the preparation of HMG-CoA reductase inhibitors with a 3,5-Dihydroxypentanoate subunit.

(57) A novel process for the preparation for intermediates in the synthesis of antihypercholesterolemic compounds of the HMG-CoA reductase type of the following general formula

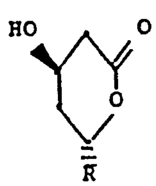

wherein R is, e.g.,

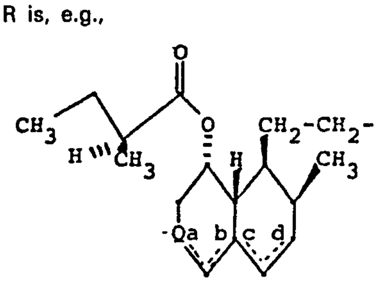

wherein Q is, e.g.,

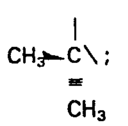

and a, b, c, and d represent, e.g., optional double bonds, involving a stereospecific reduction of a compound of the formula

$$R-\overset{O}{\overset{\|}{C}}CH_2\overset{O}{\overset{\|}{C}}CH_2CO_2(C_{1-5}alkyl)$$

with a trialkylborane and sodium borohydride.

EP 0 204 287 A2

2028S/0985A



TITLE OF THE INVENTION:

# PROCESS FOR THE PREPARATION OF HMG-CoA REDUCTASE INHIBITORS WITH A 3,5-DIHYDROXYPENTANOATE SUBUNIT

## BACKGROUND OF THE INVENTION

Hypercholesterolemia is known to be one of the prime etiological components of cardiovascular disease such as atherosclerosis, and there is still no effective antihypercholesterolemic agent available that has found wide patient acceptance. The bile acid sequestrants seem to be moderately effective but they must be consumed in large quantities, i.e. several grams at a time and they are not very palatable.

There are agents known, however, that are very active antihypercholesterolemic agents that function by limiting cholesterol biosynthesis by inhibiting the enzyme, HMG-CoA reductase. These agents include the natural fermentation products compactin and mevinolin and a variety of semi-synthetic and totally synthetic analogs

thereof. These compounds have the following general structural formula:

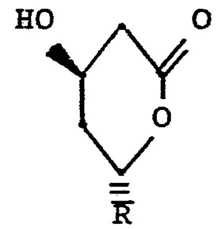

wherein R is

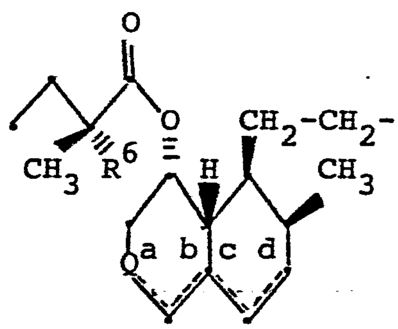

One group of totally synthetic analogs are disclosed in U.S. Patent 4,375,475 and have the same general structural formula:

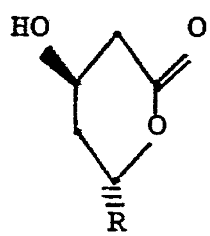

wherein R is

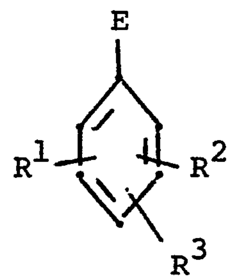

In the usual course of synthesis of these lactones an intermediate ester and dihydroxy acid are encountered:

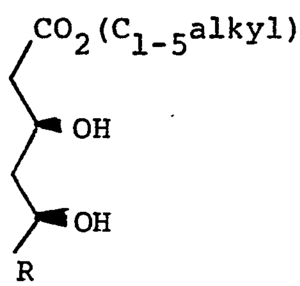

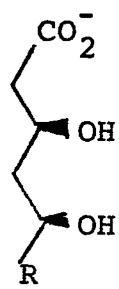

<u>ester</u> <u>dihydroxy acid</u>

Each of these entities, as well as the lactone, demonstrate antihypercholesterolemic activity <u>in vivo</u>, of comparable magnitude. However, for these compounds to manifest a useful degree of activity, it is essential that the compounds have the particular 3R:5S/3S:5R steric relationship shown in the structures.

One of the prior art synthesis of these compounds comprises reduction of ß-hydroxyketones <u>1a</u> or <u>1b</u>

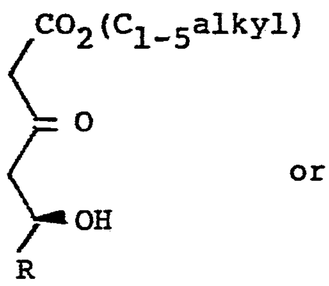

or

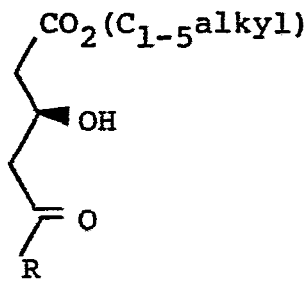

1a 1b

but the prior art methodology exhibited no stereoselectivity producing mixtures of the 3R,5R/3S,5S; and 3S,5R/3R,5S racemates in approximately 1:1 ratios. The enormously expensive procedures required to separate these diastereomers and the need to discard the unwanted half of the product made these products commercially unattractive.

Reduction of substrates of this type have been reported with sodium borohydride in U.S. Patent 4,255,444; and with zinc borohydride by Hsu et al in J. Amer. Chem. Sec., 105, 593-601 (1983); and by Narasaka et al in Chemistry Letters, 1415-1418 (1980) who disclosed the use of tri-n-butylborane and sodium borohydride at low temperature. The latter system provided considerable stereoselectivity, but in the examples given none of the substrates included other functional groups which could conceivably participate in the reductive process.

SUMMARY OF THE INVENTION

This invention relates to a novel process for the preparation of the intermediate ester in the synthesis of antihypercholesterolemic agents which contain a 4-hydroxy-3,4,5,6-tetrahydro-2H-2-pyran-2-one moiety. This process involves the stereoselective reduction of a ß-diketone intermediate of the general structural formula 2:

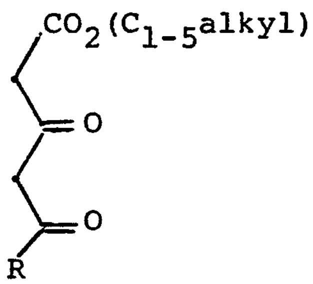

2

and its preparation.

DETAILED DESCRIPTION OF THE INVENTION

A process for the preparation of a compound represented by the following general formula (I):

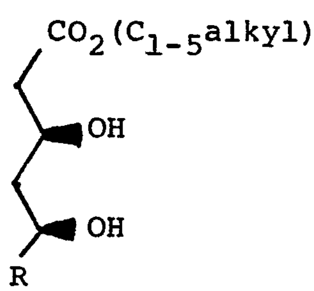

(I)

wherein R is:

(A) 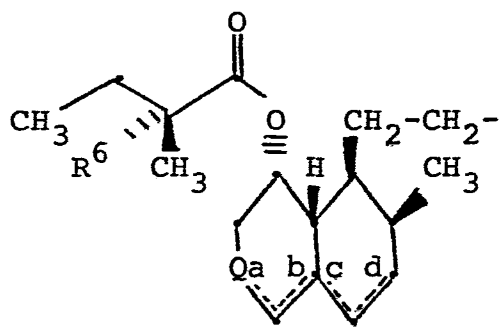

wherein Q is 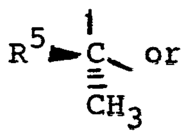 or $R^5$-CH; $R^5$ is H or OH;

$R^6$ is hydrogen or methyl; and a, b, c, and d represent optional double bonds, especially where b and d represent double bonds or a, b, c and d are all single bonds; or

(B) 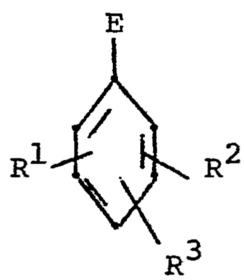

wherein E is -CH=CH- or $-CH_2CH_2-$; and $R^1$, $R^2$ and $R^3$ are each selected from
halo such as chloro, bromo or fluoro,
$C_{1-4}$alkyl,
$C_{1-4}$haloalkyl,
phenyl
phenyl with one or more substituents independently selected from

halo,

$C_{1-4}$alkyl, and

$C_{1-4}$alkoxy, or

$R^4O$ in which $R^4$ is

phenyl,

halophenyl, or

substituted phenyl-$C_{1-3}$alkyl wherein the substituents are selected from halo and $C_{1-4}$ haloalkyl;

comprises:

(1) reacting a compound of the Formula (II)

RCN (II)

wherein R is defined above, with the zinc salt of the dianion from a compound of the Formula (III)

```
   O
   ||
CH3CCH2CO2(C1-5-alkyl)   (III)
```

to afford a compound of the Formula (IV)

```
NH2 O
|   ||
RC=CHCCH2CO2(C1-5alkyl)   (IV)
```

wherein R is defined above;

(2) hydrolyzing under acidic conditions the compound of the Formula (IV) to yield a compound of the Formula (V)

```
   O     O
   ||    ||
R-CCH2CCH2CO2(C1-5alkyl)   (V)
```

wherein R is defined above; and

(3) stereoselectively reducing the compound of the Formula (V) using a tri($C_{1-4}$alkyl)borane, an activating agent and an alkali metal borohydride in an inert solvent at -30°C to -10°C followed by isolation of the compound of the Formula (I).

In a first preferred embodiment R is the radical (A). Illustrative of this embodiment are the compounds of the formula I wherein $R^5$ is H, $R^6$ is

H or $CH_3$ and b and d represent double bonds or a, b, c and d are all single bonds.

In a second preferred embodiment, R is the radical (B). Illustrative of this embodiment are the compounds of the formula I wherein E is -CH=CH-, $R^1$ is in the 6-position and represents phenyl with 1 or 2 substituents independently selected from chloro, fluoro, methyl and methoxy; and $R^2$ and $R^3$ are independently selected from halo and $C_{1-3}$ alkyl in the 2- and 4-positions.

In the most preferred embodiment, R is:

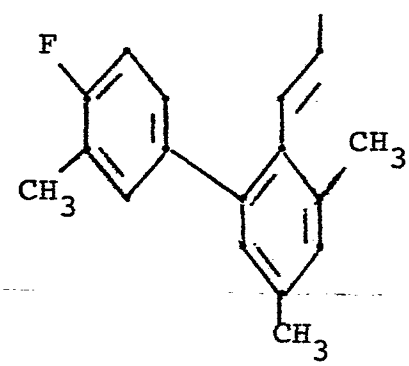

The preparation of the compound of the Formula (IV), an enamino-ketoester, is accomplished by condensing the appropriately substituted nitrile (II) with the zinc salt of an acetoacetate ester dianion under standard reaction conditions. Specifically, the zinc salt of the dianion is formed under anhydrous conditions in an aprotic solvent utilizing a non-nucleophilic base and then a zinc halide, such as zinc chloride is added. The appropriately substituted nitrile is then added at about ambient temperature and the reaction allowed to go to completion at ambient temperature.

The preparation of the compound of the Formula (V), a diketoester, utilizes a controlled acidic hydrolysis of the enamino-ketoester (IV) with an organic acid. Specifically, the enamino-ketoester is dissolved in a water miscible solvent and then an aqueous solution of the organic acid is added at ambient temperature.

The preparation of the compound of the Formula (I), a dihydroxyester, is accomplished by a stereospecific reduction of the diketoester. This process comprises the treatment of compound of the Formula (V) with between 0.1 and 2.0 molecular equivalents of a tri($C_{1-4}$alkyl) borane, such as tri-ethyl-, tri-isopropyl-, tri-n-butyl-, triisobutyl- or tri-sec-butylborane and an activating agent such as air or pivalic acid followed by the stereospecific reduction of the dialkyl borinic acid ester with 1-2 molecular equivalents of an alkali metal borohydride, such as sodium borohydride. The process is conducted in an inert solvent such as: a hydrocarbon, e.g. hexane, toluene, cyclohexane or the like; a halocarbon, e.g. methylene chloride, chloroform, ethylene dichloride or the like; a $C_{1-4}$alkanol, e.g. methanol, ethanol,isopropanol or the like; or an ether, e.g. diethyl ether, tetrahydrofuran, 1,2-dimethoxyethane or the like; or mixtures thereof. The preferred solvent is a mixture of tetrahydrofuran and methanol in a ratio of about 3-6 volumes of tetrahydrofuran to 1 volume of methanol. When a catalytic amount of a tri($C_{1-4}$alkyl) borane, i.e. less than 1.0 molecular equivalents, is utilized in the reduction, and $C_{1-4}$alkanol must be employed in the solvent to

generate the tri($C_{1-4}$alkyl) borane and increase the stereoselectivity of the reaction. The reaction is conducted at temperatures between about -30°C and -10°C, preferably at about -20°C for about 30 minutes to 3 hours.

A preferred process comprises the treatment of Compound of the Formula (V) with between 0.10 and 1.0 molecular equivalents of a tri($C_{1-4}$alkyl) borane and between 0.01 to 0.05 molecular equivalents of pivalic acid in an inert solvent and then after cooling to between -10 and -30°C adding a $C_{1-4}$ alkanol followed by the addition of 1-2 molecular equivalents of an alkali metal borohydride. Under these preferred condition greater than 95 percent of the product is in the desired stereochemical conformation.

The reaction mixture is conveniently worked up by quenching into hydrogen peroxide/water, and extracting the product into an organic solvent.

The starting materials wherein R is the radical (A) are conveniently prepared from the corresponding aldehydes employing standard chemical transformations. The aldehydes may be prepared using the synthetic methods described by Hsu et al., J. Am. Chem. Soc., 1983, 105, pp. 593-601 in the total synthesis of compactin. The starting materials wherein R is the radical (B) are known in the art.

The following examples illustrate the present invention and as such are not to be considered as limiting the invention set forth in the claims appended hereto.

## EXAMPLE 1

Preparation of tert.-butyl (E)-7-(4'-fluoro-3,3',5-trimethyl[1,1'-biphenyl]-2-yl)-5-amino-3-oxo-4,6-heptadienoate

A round-bottomed flask was charged with sodium hydride (6.37 g, 50% by weight in oil, 132.7 mmol) and sieve-dried tetrahydrofuran (20 ml), cooled to 0°C, and a solution of tert.-butyl acetoacetate (20.0 g, 126.4 mmole) in tetrahydrofuran (10 ml) was added while maintaining the temperature below 14°C. After aging for 1 hour at 0°C, the mixture was cooled to -75°C and a solution of n-butyllithium in hexane (54 ml, 124.2 mmol, 2.3 Molar) was added, keeping the temperature below -30°C. The mixture was warmed to -15°C and a solution of zinc chloride in tetrahydrofuran (100 ml, 135 mmole, 1.35 M) was added. The mixture was warmed to 17°C and E-3-(4'-fluoro-3,3',5-trimethyl[1,1'-biphenyl]-2-yl)-2-propenoic acid nitrile (20.0 g, 75.47 mmole) was added and the mixture stirred for 26 hours at 20-24°C.

The mixture was carefully quenched with water (7 ml), diluted with 1 M aqueous hydrochloric acid (100 ml) then extracted with ethyl acetate (200 ml). The ethyl acetate layer was washed with 1 M aqueous hydrochloric acid (70 ml) then water (150 ml), dried over sodium sulfate and filtered. The solution was concentrated to dryness, *in vacuo*. The residue was diluted with hexanes (150 ml) heated to 60°C then aged with stirring at ambient temperature overnight. After cooling to 0°C the mixture was filtered and dried *in vacuo* to give the title compound as a yellow solid.

EXAMPLE 2

Preparation of tert.-butyl (E)-7-(4'-fluoro-3,3',5-trimethyl[1,1'-biphenyl]-2-yl)-3,5-dioxo-4,6-heptadienoate

The compound of Example 1 (4.25 g, 10.03 mmole) was dissolved in tetrahydrofuran (25 ml) and a solution of 88% formic acid (25 ml) in water (10 ml) was added. The mixture was stirred at 20-25°C for 40 hours then neutralized at 15°C with saturated aqueous sodium bicarbonate solution. The mixture was extracted with ethyl acetate, the organic extract washed with water then dried over sodium sulfate. After filtration, the solution was concentrated to provide the title compound as a clear, colorless oil.

EXAMPLE 3

Preparation of tert.-butyl (E)-7-(4'-fluoro-3,3',5-trimethyl[1,1'-biphenyl]-2-yl)-3,5-dihydroxy-6-heptenoate

To a solution of the compound of Example 2 (424 mg, 1.0 mmol) in tetrahydrofuran under nitrogen was added pivalic acid (5 mg, 5 mole percent) and triethylborane (1.0 ml of a 1.06 M solution in heptane, 1.06 mmol). After stirring for one hour at ambient temperature, tetrahydrofuran (4.0 ml) was added and the reaction mixture cooled to -25°C. Methanol (1.5 ml) was added dropwise and the sodium borohydride (151 mg, 4.0 mmol) was added in three portions over two hours while maintaining the temperature below -20°C. The reaction was quenched by pouring the reaction mixture into 30 percent aqueous hydrogen peroxide (20 ml) and then extracted with ethyl acetate. The organic phase was washed

with water, dried over anhydrous sodium sulfate and concentrated in vacuo to afford the desired product with a 96 percent of the desired stereoisomers.

## EXAMPLES 4 to 13

Utilizing the general procedures of Examples 1, 2, and 3, the following compounds of the Formula I are prepared from the appropriate starting materials.

| Compound Number | $R^1$ |
|---|---|
| 4 | 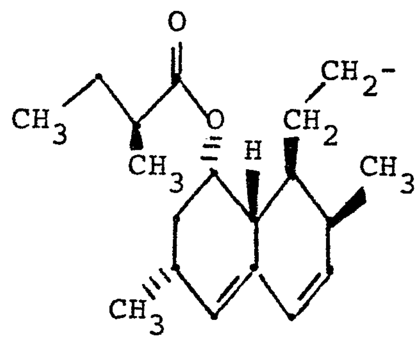 |
| 5 | 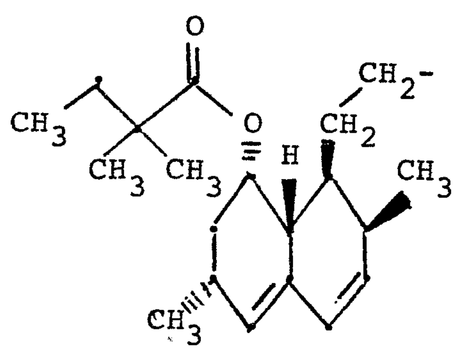 |

| Compound Number | $R^1$ |
| --- | --- |
| 6 | 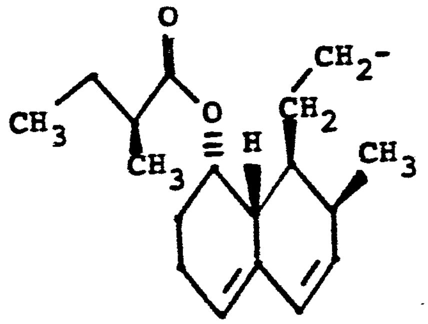 |
| 7 | 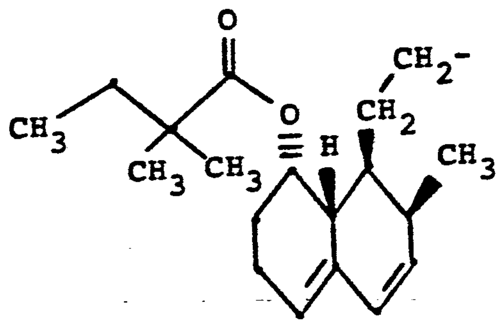 |
| 8 | 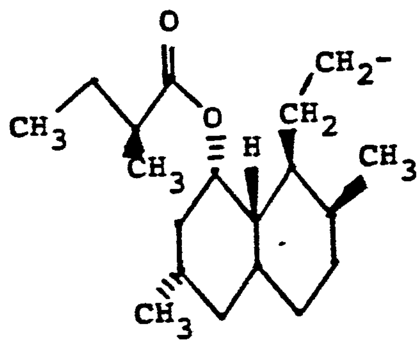 |
| 9 | 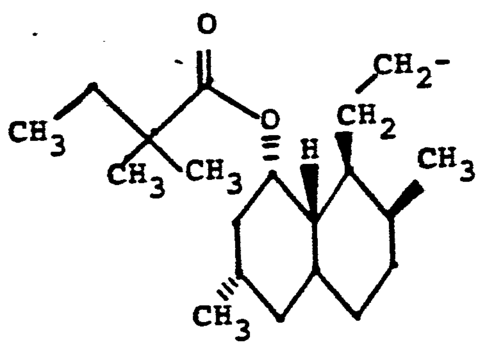 |

| Compound Number | $R^1$ |
|---|---|
| 10 | 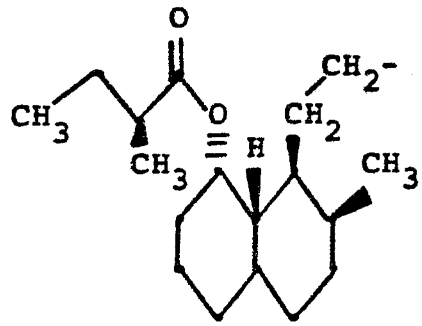 |
| 11 | 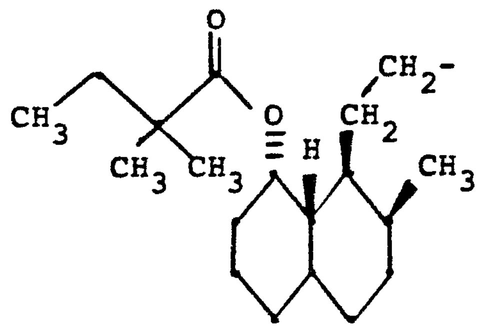 |
| 12 | 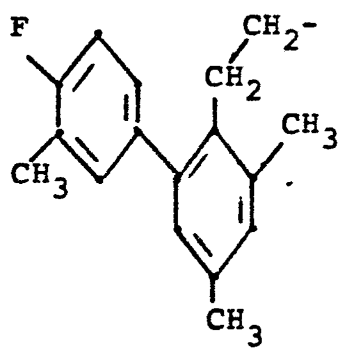 |
| 13 | 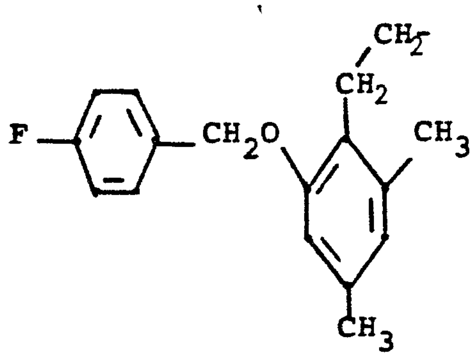 |

WHAT IS CLAIMED:

1. A process for the preparation of a compound represented by the following general formula (I):

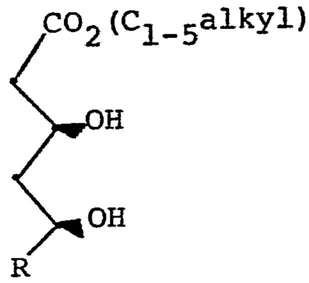

(I)

wherein R is:

(A) 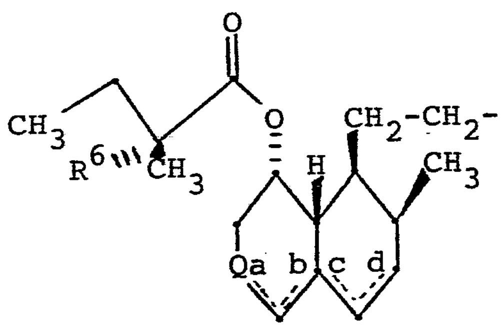

wherein Q is 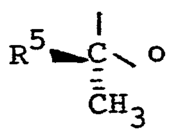 or 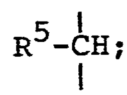; $R^5$ is H or OH;

$R^6$ is hydrogen or methyl; and a, b, c, and d represent optional double bonds, especially where b and d represent double bonds or a, b, c and d are all single bonds; or

(B) 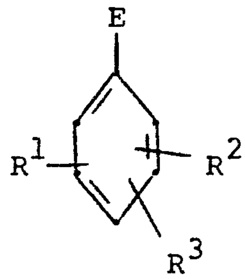

wherein E is -CH=CH- or $-CH_2CH_2-$; and $R^1$, $R^2$ and $R^3$ are each selected from
halo such as chloro, bromo or fluoro,
$C_{1-4}$alkyl,
$C_{1-4}$haloalkyl,
phenyl
phenyl with one or more substituents independently selected from
- halo,
- $C_{1-4}$alkyl, and
- $C_{1-4}$alkoxy, or

$R^4O$ in which $R^4$ is
- phenyl,
- halophenyl, or
- substituted phenyl-$C_{1-3}$alkyl wherein the substituents are selected from halo and $C_{1-4}$ haloalkyl;

which comprises:
stereoselectively reducing the compound of Formula (V)

$$R-\overset{O}{\overset{\|}{C}}CH_2\overset{O}{\overset{\|}{C}}CH_2CO_2(C_{1-5}alkyl) \quad (V)$$

wherein R is defined above, using a tri($C_{1-4}$alkyl)-borane, an activating agent and an alkali metal borohydride in an inert solvent at -30°C to -10°C followed by isolation of the compound of the Formula (I).

2. The process of Claim 1 wherein between 0.1 and 2.0 molecular equivalents of the tri-($C_{1-4}$alkyl) borane, and between 1.0 and 2.0 molecular equivalents of the alkali metal borohydride is utilized and the inert solvent is selected from a hydrocarbon, a halocarbon, a $C_{1-4}$ alkanol and an ether and mixtures thereof.

3. The process of Claim 2 wherein between 0.8 and 1.5 molecular equivalents of the tri ($C_{1-4}$ alkyl) borane is utilized.

4. The process of Claim 2 wherein the activating agent is pivalic acid.

5. The process of Claim 1 where R is the radical (B).

6. The process of Claim 5 wherein, E is -CH=CH-, $R^1$ is in the 6-position and represents phenyl with 1 or 2 substituents independently selected from chloro, fluoro, methyl and methoxy; and $R^2$ and $R^3$ are independently selected from halo and $C_{1-3}$alkyl in the 2- and 4-position.

7. The process of Claim 6 for the preparation of (E)-7-(4'-fluoro-3,3',5-trimethyl [1,1'-biphenyl]-2-yl)-dihydroxy-6-heptenoate as a racemic mixture of the 3S,5R and 3R,5S isomers.